Europäisches Patentamt

European Patent Office

Office européen des brevets

(19)

(11) Publication number: **0 286 700**
**A1**

## EUROPEAN PATENT APPLICATION

(21) Application number: **87105425.0**

(22) Date of filing: **11.04.87**

(51) Int. Cl.4: **C12N 15/00 , C07K 13/00 , A61K 37/02 , C12P 21/00**

Claims 11 - 23 are deemed to be abandoned due to non-payment of the claims fees (Rule 31 (3) EPC).

(43) Date of publication of application:
**19.10.88 Bulletin 88/42**

(84) Designated Contracting States:
**GB**

(71) Applicant: **Kishimoto, Tadamitsu, Prof. Division of Cellular Immunology Institute for Molecular and Cellular Biology University Osaka**
**1-3, Yamadaoka Suita Osaka 565(JP)**

(72) Inventor: **Kishimoto, Tadamitsu, Prof. Dr.**
**3-5-31, Nakano Tondabayashi**
**Osaka 584(JP)**
Inventor: **Suemura, Masaki, Dr.**
**9-5, Himemuro Ikeda**
**Osaka 563(JP)**
Inventor: **Kikutani, Hitoshi, Dr.**
**2-17-B504, Senriyama-Higashi Suita**
**Osaka 565(JP)**
Inventor: **Barsumian, Edward L., Dr.**
**3-11-5-503, Senba-Nishi Mino**
**Osaka 562(JP)**

(74) Representative: **Laudien, Dieter, Dr. et al**
**Boehringer Ingelheim Zentrale GmbH ZA Patente Postfach 200**
**D-6507 Ingelheim am Rhein(DE)**

(54) **Biologically active recombinant human soluble Fc R-fragment, plasmids encoding this Fc R-fragment and the preparation thereof.**

(57) The object of the present invention is a novel recombinant water-soluble human $Fc_\epsilon R$-fragment having at least one O-glycosylation site, preferably a fragment accompanied by native O-glycosylation, which is thereby biologically active, novel plasmids encoding for said soluble $Fc_\epsilon R$, the DNA-sequences containing these novel plasmids, and the preparation thereof.

## Biologically active recombinant human soluble Fc$_\epsilon$R-fragment, plasmids encoding this Fc$_\epsilon$R-fragment and the preparation thereof

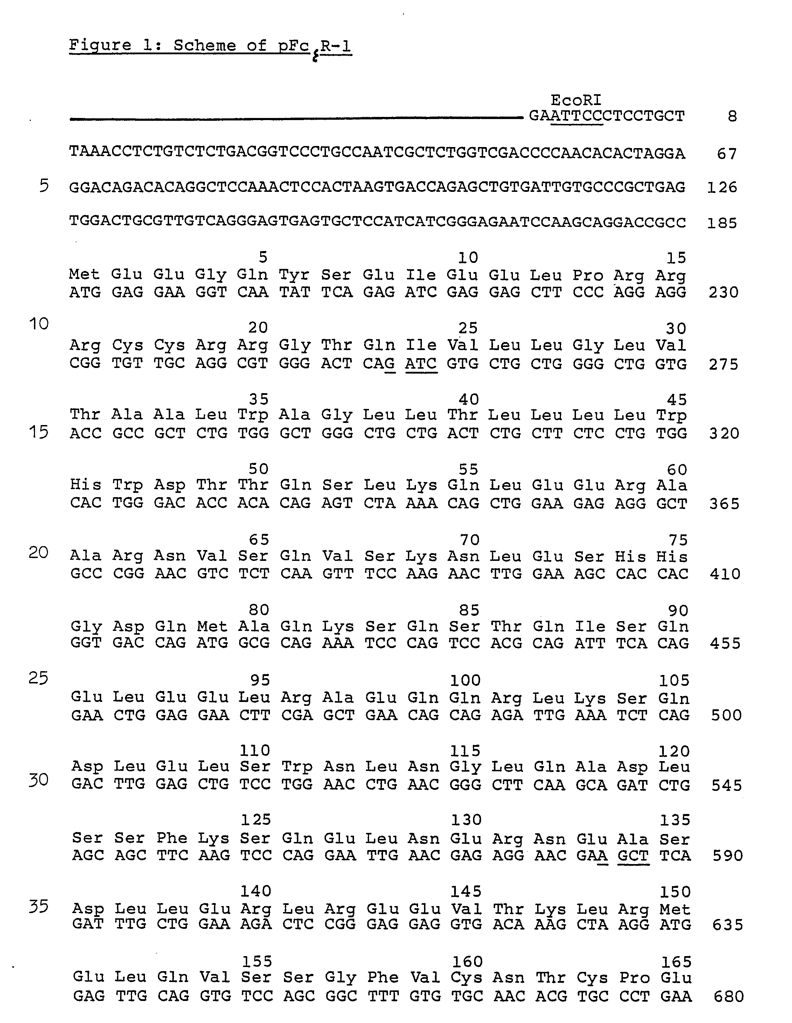

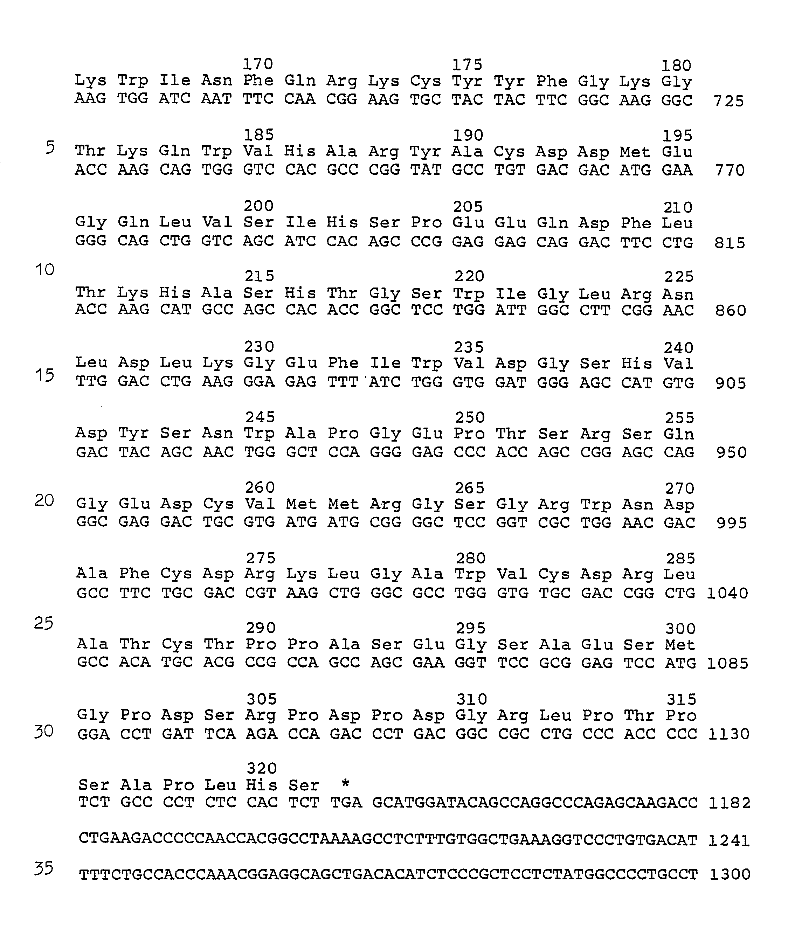

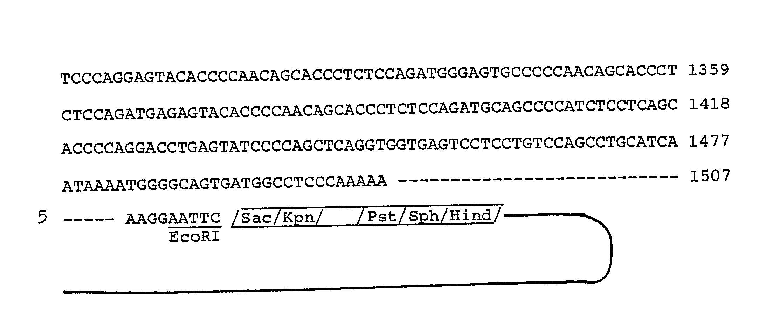

In Cell 47, 657-665 (1986) is described the molecular structure of human lymphocyte receptor for Immunoglobulin E and a cDNA clone encoding said Fc$_\epsilon$R, which consist of 321 amino acids without any signal sequence. This molecule is unusual in that it is oriented with its N-terminus in the cytoplasm side and its C-terminus located outside the cell. Moreover, from the publication in Cell it is known that the native water-soluble part of Fc$_\epsilon$R is a product of proteolytic cleavage between amino acids 149 and 150 (Arg and Met) of membrane-bound receptor (see Figure 1: scheme of pFc$_\epsilon$R-I).

It is generally accepted, that although the function of glycosylation in human proteins is not known, it has been observed that, generally speaking, a recombinant, non-glycosylated protein will have the same biological activity as the natural glycosylated molecule, for instance the glycosylated interferons are undistinguishable in biological activity with the non-glycosylated.

We have found that, unexpectedly, while glycosylated Fc$_\epsilon$R water-soluble fragment has IgE binding activity, the nonglycosylated Fc$_\epsilon$R water-soluble fragment does not demonstrate such activity. Thus for pharmacological activity e.g. in treating allergic reactions, it is necessary to ensure that recombinant product is O-glycosylated.

In addition, we have found that the membrane spanning region of the Fc$_\epsilon$R does not function as a signal sequence in the usual recombinant processes and therefore secretion of the water-soluble receptor protein from a suitable host, it is necessary to use an appropriate eucaryotic signal sequence. Such signal sequence may be provided in addition and in a position in front of the cDNA coding for the water-soluble part.

According to one aspect of the present invention therefore, we provide a novel recombinant water-soluble human Fc$_\epsilon$Rfragment having at least one O-glycosylation site, preferably a fragment accompanied by native O-glycosylation, which is thereby biologically active, and the production thereof.

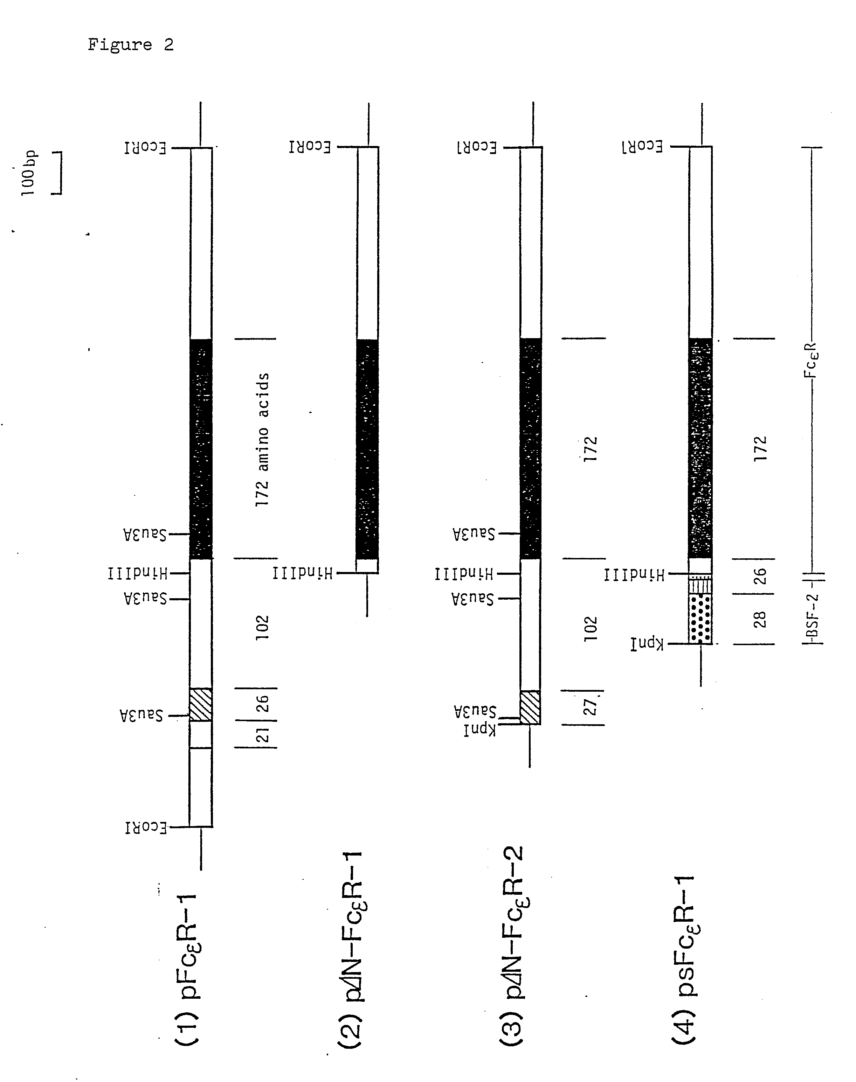

A further aspect of the present invention comprises the novel plasmids encoding for said soluble Fc$_\epsilon$R-fragment, the DNA-sequences containing these novel plasmids, and the preparation thereof (see e.g. Figure 2: schemes of pFc$_\epsilon$R-I and ps Fc$_\epsilon$R-I).

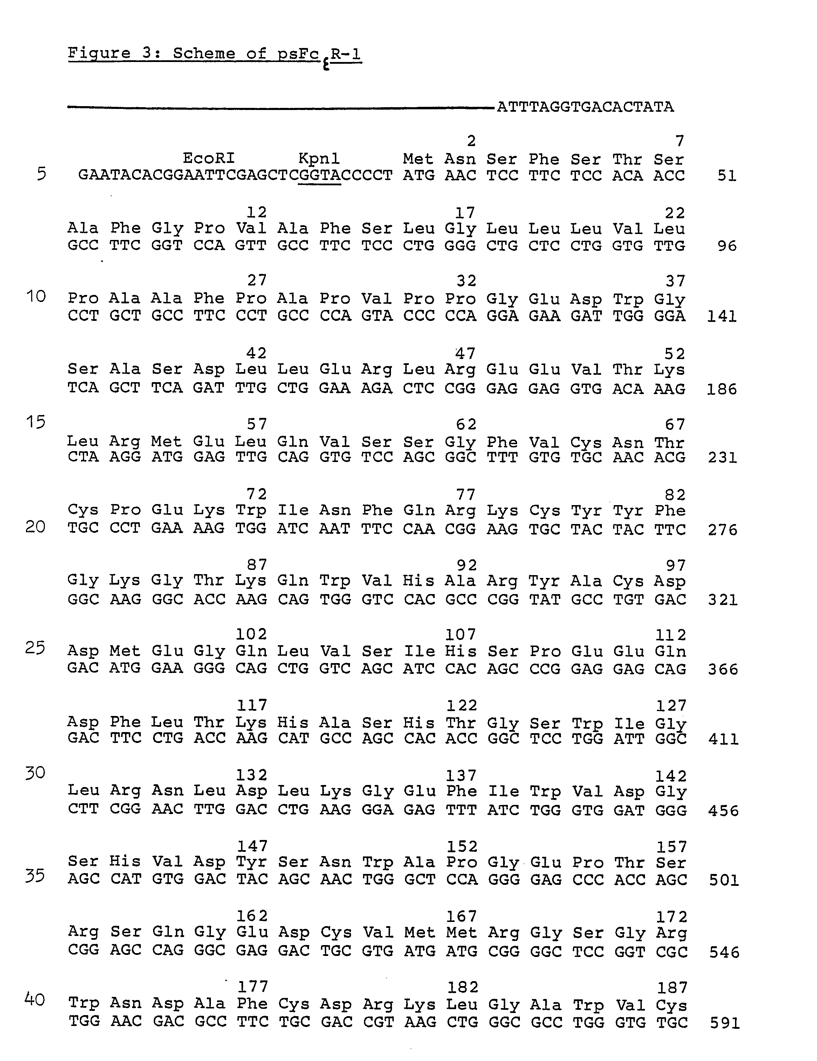

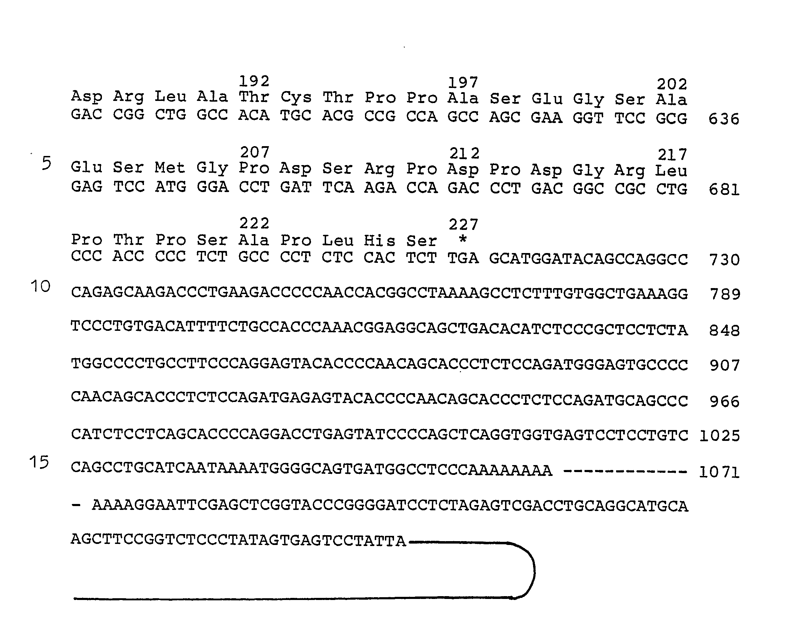

A preferred O-glycosylated water-soluble Fc$_\epsilon$R-fragment contains the amino acids 150 to 321 as shown in Figure 1, which are identical to the amino acids 55 to 226 as shown in Figure 3.

In a novel cDNA-sequence according to the present invention, the cDNA for at least part of the amino acids 1 to 148 of the Fc$_\epsilon$R, in particular the coding sequence for the N-terminal cytoplasmic region is absent so that e.g. only the coding sequence for the insoluble membrane protein and the portion encoding the water-soluble part of the cDNA of the whole receptor is present.

In this novel cDNA-sequence at least a part of the cDNA sequence coding for the amino acids 1 to 148 is replaced by a suitable cDNA fragment coding for an eucaryotic signal sequence using suitable restriction endonucleases and ligases.

For example, a plasmid containing a cDNA insert encoding the Fc$_\epsilon$R is modified by replacing at least a part of the coding sequence for the amino acids 1 to 148 e.g. the amino acids 1 to 134 by an eucaryotic cDNA signal sequence e.g. an interleukin cDNA signal sequence e.g. by the BSF-2 signal sequence. Thus, in the example described below a corresponding plasmid e.g. plasmid LE 392 deposited on August 01, 1986 under number FERM BP-1116 (Fermentation Research Institute, Agency of Industrial Science and Technology, Japan - see also European Patent Application 86 111 581.4, filed on August 21, 1986) or pGEM4 (pFc$_\epsilon$R-I) (see Figure 1) described in Cell 47, 659 (1986) was digested with HindIII, whereby a 1.0 kbp HindIII-fragment was obtained containing the coding sequence for the amino acids 134 to 321 of the full-length Fc$_\epsilon$R cDNA. The recessed 3'-ends of this fragment were then filled in with the Klenow fragment of DNA polymerase and the DNA subsequently digested with PstI. The obtained fragment was then cloned in a suitable vector, preferably with a BamHI-PstI digested pBSF2-L8. The vector is conveniently prepared as follows:

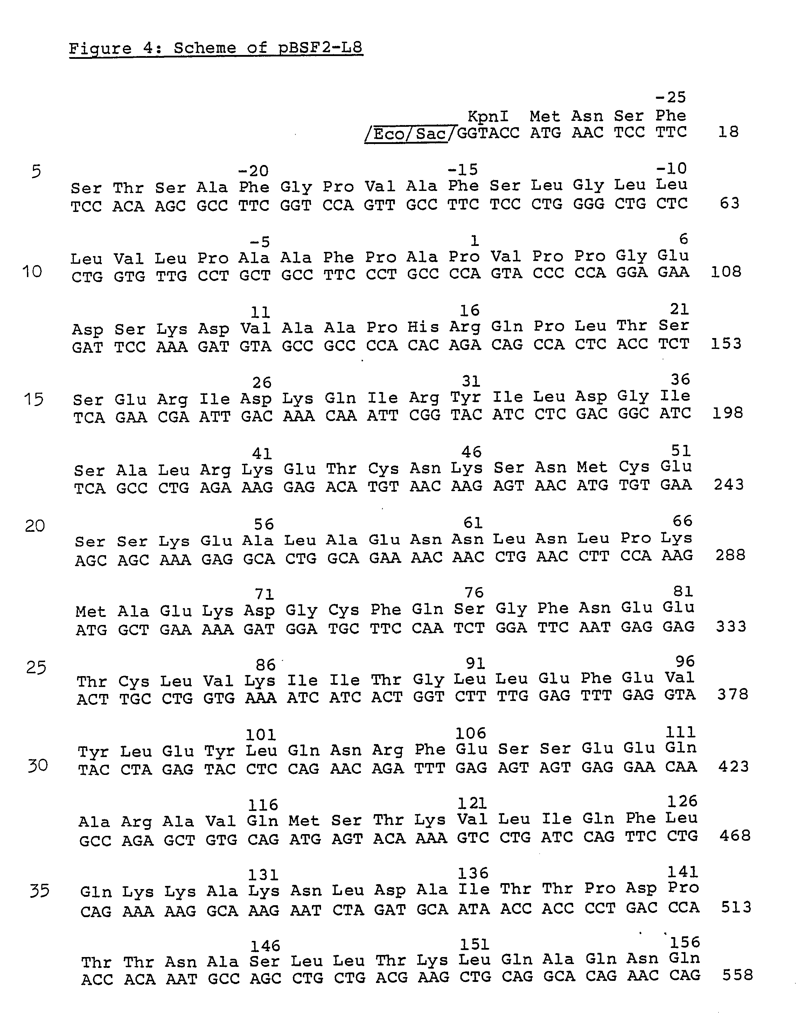

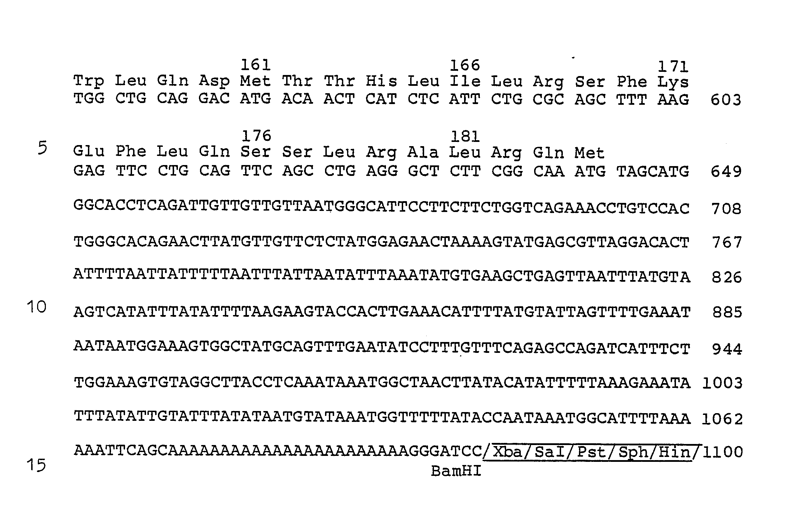

The EcoRI-BamHI 1,2 kbp BSF-2 cDNA insert was prepared by digestion of pBSF-2.38 (see Nature 324, 73-76 (1986)) with HindIII and BamHI. The obtained fragment containing a full length BSF-2 cDNA was then digested with HinfI and the recessed 3'-end filled in with Klenow fragment of DNA polymerase. After KpnI digestion, the obtained KpnI-HinfI 110 bp fragment containing the BSF-2 leader sequence was cloned into the multiple cloning site of pGEM4 digested previously with KpnI and SmaI. One of the selected clones was propagated and named as pBSF2-L8 (see Figure 4).

pBSF2-L8 was digested with BamHI and the recessed 3'-ends filled in with Klenow fragment of DNA polymerase. After the filling in of the BamHI site, the above mentioned HindIIIPstI Fc$_\epsilon$R cDNA was cloned into BamHI-PstI digested pBSF2-L8 as mentioned hereinbefore. One of the selected clones was propagated and named as psFc$_\epsilon$R-I (see Figure 3).

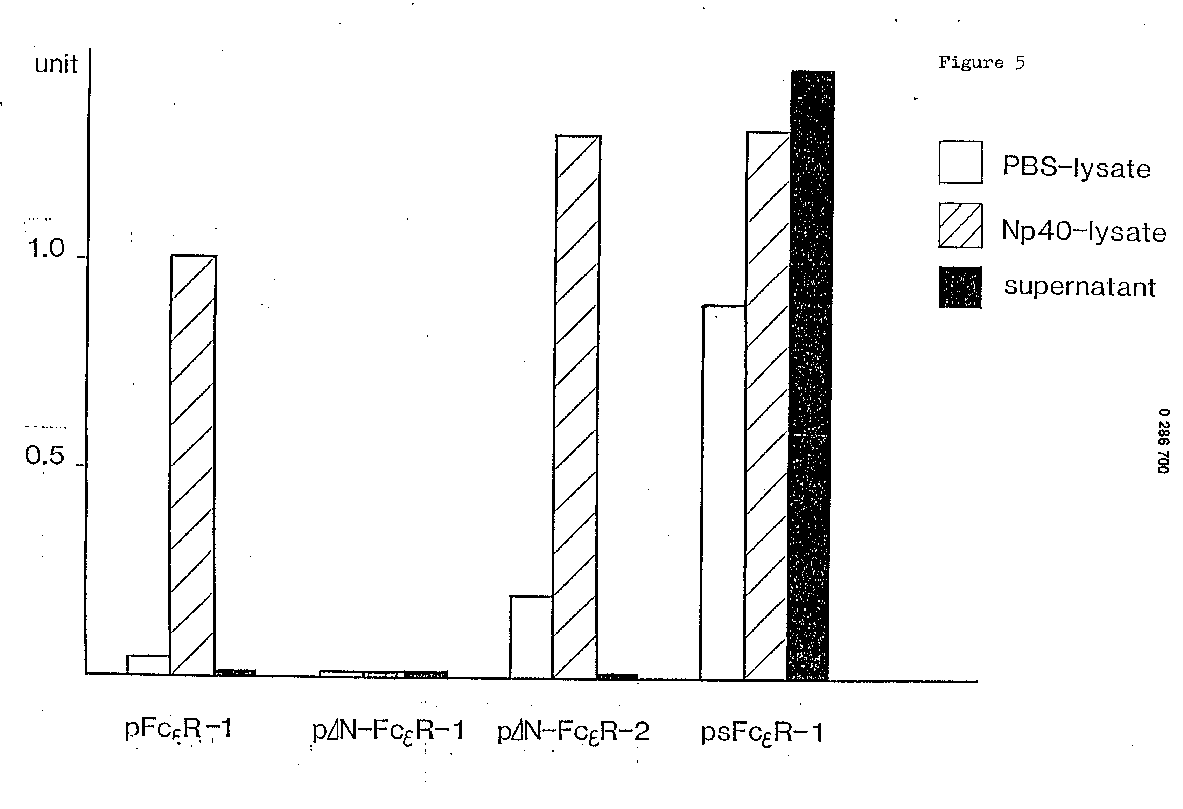

To compare the biological activity of the proteins produced by the clones pFc$_\epsilon$R-I, psFc$_\epsilon$R-I, pΔNFc$_\epsilon$R-I and pΔNFc$_\epsilon$R-2 (see Figure 2) these plasmids were linearized by digestion with the appropriate enzymes, e.g. pFc$_\epsilon$R-I and psFc$_\epsilon$R-I with BamHI and pΔNFc$_\epsilon$R-I and pΔNFc$_\epsilon$R-2 with EcoRI, and the obtained fragments are used as a template to synthesize mRNA with SP6 RNA polymerase. The resulting mRNA's were injected into Xenopus leavis oocytes. After 2 days of incubation, the Fc$_\epsilon$R activities in the culture supernatants and the lysates of oocytes are determined by an enzyme linked immunosorbent assay (ELISA) utilizing anti-Fc$_\epsilon$R antibodies 3-5 and 8-30 (see European Patent Application 86 III 488.2, filed on August 19, 1986), which recognize two different epitopes on Fc$_\epsilon$R. As shown in Figure 5 Fc$_\epsilon$R activity was determined for the NP-40 lysate of oocytes, in PBS-lysate of oocytes and in oocyte culture supernatant. It will be seen that whereas no activity could be detected in PBS-lysate and culture supernatant of oocytes injected with transcripts of pFc$_\epsilon$R-I, pΔNFc$_\epsilon$R-I and pΔNFc$_\epsilon$R-2 Fc$_\epsilon$R activity was detected in supernatants and PBS-lysates after injection with fragments of psFc$_\epsilon$R-I.

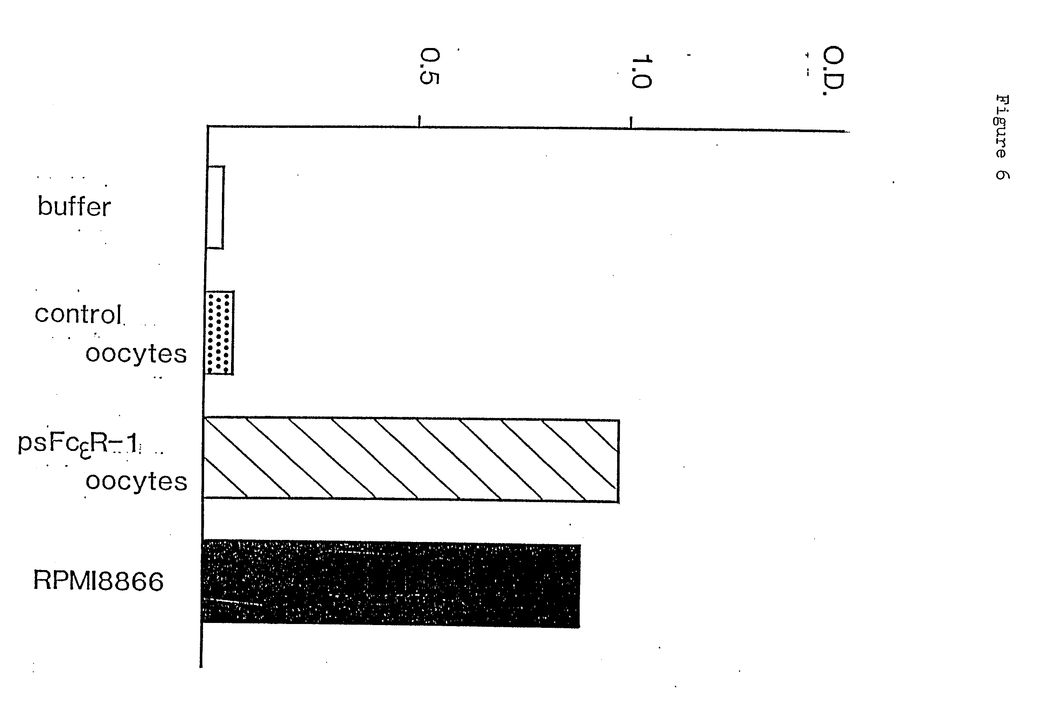

Furthermore, we determined that the Fc$_\epsilon$R water-soluble fragment secretion product from oocytes have the properties of binding IgE by means of a modified ELISA using anti-Fc$_\epsilon$Rantibody 3-5, IgE and AP-anti-human IgE: The culture supernatant from the oocytes injected with psFc$_\epsilon$R-I mRNA was incubated on 3-5 antibody-coated plates which were then incubated with human IgE and finally with AP-anti-IgE. The results established clearly that Fc$_\epsilon$R secreted from the oocytes formed a complex with IgE (see Figure 6). Binding with non-transformed oocyte supernatant, buffer, and RPMI 8866 supernatant were carried out as a control.

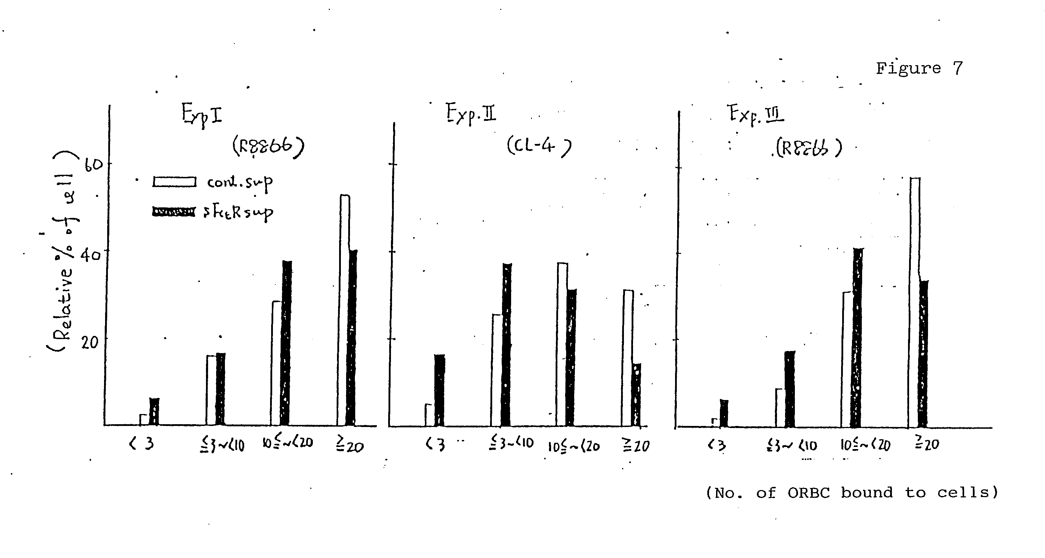

In order to evaluate the IgE-binding property of the soluble Fc$_\epsilon$R fragment derived from oocytes injected with psFc$_\epsilon$R-I mRNA, the oocyte supernatant was further tested for its ability to inhibit the rosette formation between ORBC coated with human IgE and SKW6-CL4 cells bearing on their surface Fc$_\epsilon$R: The presence of soluble Fc$_\epsilon$R reduced the number of rosette forming cells to which more than 20 ORBC were bound indicating that the soluble receptor is competing with the cell membrane Fc$_\epsilon$R for the IgE bound on the surface of ORBC (see Figure 7).

The corresponding genes containing an eucaryotic signal sequence and at least the coding sequence for the amino acids 150 to 321 of the full-length Fc$_\epsilon$R cDNA can be introduced into organisms under conditions which lead to high yields thereof, as mentioned hereinbefore. Useful hosts and vectors are well known to those of skill in the art.

In general, eukaryotic microbes, such as yeast cultures can be used for expression. For example, Saccharomyces cerevisiae is most commonly used among eukaryotic microorganisms, although a number of other species are commonly available. For expression in Saccharomyces, the plasmid YRp7, for example (Stinchcomb, et al., Nature 282, 39, (1979); Kingsman et al., Gene 7, 141 (1979); T-schumper, et al., Gene 10, 157 (1980)) and plasmid YEpI3 (Bwach et al., Gene 8, 121-133 (1979)) are commonly used. The plasmid YRp7 contains the TRPI gene which provides a selection marker for a mutant strain of yeast lacking the ability to grow in tryptophan, for example ATCC No. 44076. The presence of the TRPI lesion as a characteristic of the yeast host cell genome then provides an effective environment for detecting transformation by growth in the absence of tryptophan. Similarly, the plasmid YEpI3 contains the yeast LEU2 gene which can be used for complementation of a LEU2 minus mutant strain.

Suitable promoting sequence in yeast vectors include the 5'-flanking region of the genes for ADH I (Ammerer, G., Methods of Enzymology 101, 192-201 (1983)), 3-phosphoglycerate kinase (Hitzemann, et al., J. Biol. Chem. 255, 2073 (1980)) or other glycolytic enzymes (Kawasaki and Fraenkel, BBRC 108, 1107-1112 (1982)), such as enolase, glyceraldehyde-3-phosphate dehydrogenase, hexokinase, pyruvate decarboxylase, phosphofructokinase, glucose-6-phosphate isomerase, phosphoglucose isomerase, and glucokinase. In constructing suitable expression plasmids, the termination sequences associated with these genes are also ligated into the expression vector 3' to the sequence to be expressed, to provide polyadenylation of the mRNA and termination.

Other promotors, which have the additional advantage of transcription controlled by growth conditions are the promotor regions of the genes for alcohol dehydrogenase-2, isocytochrome C, acid phosphatase, degradative enzymes associated with nitrogen metabolism, the aforementioned glyceraldehyde-3-phosphate dehydrogenase, and enzymes responsible for maltose and galactose utilization. Promotors which are regulated by the yeast mating type locus, such as the promotors of the genes BARI, MR-alpha-I, STE2, STE3, STE5 can be used for temperature regulated system by using temperature dependent siv mutations (Rhine, Ph. D. Thesis, University of Oregon, Eugene, Oregon (1979), Herskowitz and Oshima in The Molecular Biology of the Yeast Sacharomyces, Part I, 181-209 (1981), Cold Spring Harbor Laboratory). These mutations directly influence the expressions of the silent mating type cassettes of yeast, and therefore indirectly the mating type dependent promotors.

Generally, however, any plasmid vector containing a yeast compatible promotor, origin of replication and termination sequences is suitable.

In addition to microorganisms, cultures of cells derived from multicellular organisms may also be used as hosts. In principle, any such cell culture is applicable, whether from vertebrate or invertebrate cell cultures. However, interest has been greatest in vertebrate cells, and propagation of vertebrate cells in culture (tissue culture) has become a routine procedure in recent years (Tissue Culture, Academic Press, Kruse and Patterson, Editors (1973)). Examples of such useful host cell lines are VERO and HeLa cells, Chinese hamster ovary (CHO) cell lines, and WI38, BHK, COS-7 and MDCK cell lines. Expression vectors for such cells usually include (if necessary) an origin of replication, a host specific or viral promotor located in front of the gene to be expressed, along with any necessary ribosome binding sites, RNA splice sites, polyadenylation site, and transcriptional terminator sequences.

For use in mammalian cells, the control functions on the expression vectors are often provided by viral material. For example, commonly used promotors are derived from polyoma, Adenovirus 2, and most frequently Simian Virus 40 (SV40). The early and late promotors of SV40 are particularly useful because both are obtained easily from the virus as a fragment which also contains the SV40 viral origin of replication (Fiers et al., Nature 273, 113 (1978)). Smaller or larger SV40 fragments may also be used, provided they include the approximately 250 bp sequence extending from the Hind III site toward the Bgl I site at the viral origin of replication. It is also possible, and often desirable, to utilize promotor or control sequences normally associated with the desired gene sequence, provided such control sequences are compatible with the host cell systems.

An origin of replication may be provided either by onstruction of the vector to include an exogenous origin, derived from SV40 or other viral (e.g., Polyoma, Adeno, VSV, BPV, etc.) sources, or may be provided by the host cell chromosomal replication mechanism. If the vector is integrated into the host cell chromosome, the latter is often sufficient.

The prepared biologically active recombinant water-soluble human Fc$_\epsilon$-receptor prepared according to the invention is suitable for the treatment of local and allergic reactions induced by expression of IgE and may be incorpcrated in the suitable pharmaceutical compositions such as solutions or sprays.

The plasmids p$\Delta$NFc$_\epsilon$R-I and p$\Delta$NFc$_\epsilon$R-2 used as comparison plasmids (see Figure 5) were prepared as follows:

The plasmid LE 392 deposited on August 0l. l986 under number FERM BP-lll6 (Fermentation Research Institute, Agency of Industrial Science and Technology, Japan - see also European Patent Application 86 lll 58l.4, filed on August 2l, l986) or pGEM4 (pFc$_\epsilon$R-l) (see Figure l) described in Cell 47, 659 (l986) was digested with HindIII and EcoRI. The isolated cDNA-fragment starting with the nucleotide 584 as shown in Figure l was cloned into a HindIII and EcoRI digested pGEM4. One of the selected clones is propagated and named as p$\Delta$NFc$_\epsilon$R-l.

The above mentioned plasmid LE 392 or pGEM4 (pFc$_\epsilon$R-l) was digested with EcoRI, and a fragment containing the fulllength ~l.7 Kbp Fc$_\epsilon$R cDNA obtained. The obtained EcoRI-fragment was then partially digested with Sau3A to remove the cDNA sequence encoding for the putative cytoplasmic domain, e.g. for the amino acids l to 23 and a cDNA-fragment starting with the nucleotide 254 as shown in Figure l obtained, which was ligated with a palyndoromic 26mer linker of formula

5'-GATCTGAGTCATGGTACCATGACTCA-3'

to restore an ATG start codon at the 5'-end and a KpnI restriction site. The thus obtained ligated fragment was digested with KpnI and cloned into KpnI and EcoRI digested pGEM4. Only those clones were selected by colony hybridiza tions wherein the region for the putative cytoplasmic domain was missing. One clone was selected and propagated and named as p$\Delta$NFc$_\epsilon$R-2.

The following examples, which are not exhaustive, will illustrate the invention in greater detail:

General Materials and Methods:

The monoclonal anti-Fc$_\epsilon$R antibodies 3-5 ($\gamma_1$) and 8-30 ($\mu$) were produced by hybridization of P3Ul myeloma with spleen cells from Balb/c mice immunized with RPMI-8866 cells (see European Patent Application No. 86 ll0 420.6 of the same applicant, filed on July 29, l986). The 8-30 antibody recognizes the epitope close to the IgE binding site of Fc$_\epsilon$R and could block binding of IgE to 8866 lymphoblastoid cells. The 3-5 antibody, recognizes a different epitope on Fc$_\epsilon$R and can not block efficiently IgE binding to its receptors. These antibodies precipitate 46 kd and 25 kd polypeptides under reducing and non reducing conditions. The monoclonal antibodies were purified from ascites by 50 % saturated ammonium sulfate precipitation followed by gel filtration using Sepharose 6B (Pharmacia Fine Chemical, Uppsala, Sweden) for IgM class or ion exchange chromatography using QAE-Sephadex (Pharmacia Fine Chemical) for IgGl. The polyclonal mouse IgG was isolated in the same fashion. The anti-mouse IgM-alkaline phosphatase conjugate was purchased from Tago (Burlingame, CA).

## Example A

### Preparation of the fragments encoding a water-soluble part of $Fc_\epsilon R$

The plasmid LE392 deposited in E.coli on August 0I, I986 under number FERM BP-III6 (Fermentation Research Institute, Agency of Industrial Science and Technology, Japan) according to the convention of Budapest which is a pGEM4 vector was digested with HindIII to obtain the HindIII fragment containing a region of soluble $Fc_\epsilon R$-cDNA or with HindIII-EcoRI to obtain the HindIII-EcoRI fragment containing approximately I kbp (see Figure I: starting nucleotide 584).

## Example B

### Construction of plasmid $p\Delta NFc_\epsilon R$-I

I0 μg of $pFc_\epsilon R$-I DNA were digested with 20 units of HindIII in 50 μl of a medium salt buffer (I0 mM Tris-HCl, pH 7.5, I0 mM $MgCl_2$, I mM DTT) for I hr, followed with 20 units of EcoRI in I00 μl of a high salt buffer (I00 mM NaCl, 50 mM Tris-HCl, pH 7.5, I0 mM $MgCl_2$, I mM DTT) for I hr to obtain the HindIII-EcoRI fragment containing a I kbp region of soluble $Fc_\epsilon R$-cDNA (see Figure I: starting nucleotide 584). The digested DNA was applied on a I % preparative agarose electrophoresis and the approximately I kbp HindIII-EcoRI fragments were electroeluted from minced gels and precipitated in 70 % ethanol at -80°C for I hr. I μg of pGEM4 (Promega Biotec) was digested with 2 units HindIII and 2 units EcoRI as described above, phenol-extracted and ethanol-precipitated at -80°C for I hr. The HindIII-EcoRI fragment and HindIII-EcoRI digested pGEM4 were incubated with 200 units T4 ligase in I0 μl of a ligation buffer (50 mM Tris-HCl, pH 7.4, I0 mM $MgCl_2$, I0 mM DTT, I mM spermidine, I mM ATP, 0,I mg/ml BSA) at 4°C for I6 hrs and transfected into E.coli (MCI065). One clone was selected, propagated and after confirmation of its construction named as $p\Delta NFc_\epsilon R$-I.

## Example C

### Construction of plasmid $p\Delta NFc_\epsilon R$-2

200 μg of $pFc_\epsilon R$-I were digested with 400 units EcoRI in 200 μl of a high salt buffer for 2 hrs at 37°C and were subjected to electrophoresis on a I % preparative agarose gel. Approximately I.7 kb EcoRI fragment was electroeluted and ethanol-precipitated at -80°C for I hr. 4 μg of the fragment were digested with I0 units of AccI at 37°C for 3 hrs in 40 μl of a low salt buffer and partially digested with 0.4 unit Sau3A at 37°C for 20 min, phenol-extracted and ethanol-precipitated to obtain the Sau3A-EcoRI fragment (see Figure I: starting nucleotide 254). The DNA fragment was ligated to a I.7 μg synthetic linker (5′-GATCTGAGTCATGGTACCATGACTCAGATCGTG-CTG-3′) with 200 units of T4 ligase in I0 μl of a ligation buffer at 4°C for I6 hrs, then phenol-extracted and ethanol-precipitated. The fragments were dissolved, digested with 24 units KpnI in 40 μl of a low salt buffer at 37°C for 3 hrs, phenol-extracted and ethanol-precipitated. The excess linkers were removed by gel chromatography with a Biogel A50m column. The fragments were ethanol-precipitated.

Separately, I μg of pGEM4 was digested with 8 units KpnI in 20 μl of a low salt buffer at 37°C for 2 hrs, followed by incubation with 8 units EcoRI in 40 μl of a high salt buffer at 37°C for 2 hrs.

The fragments which had been previously ligated to synthesis linkers were then ligated to the KpnI-EcoRI-digested pGEM4 by incubating with 200 units of T4 ligase in I0 μl of a ligation buffer at 4°C for I6 hrs and transfected into E.coli (MCI065). Using colony hybridization technique, the clone, $p\Delta NFc_\epsilon R$-2, which lacks only a cytoplasmic domain, was isolated and propagated.

## Example I

### Construction of plasmid $psFc_\epsilon R$-I

a) 350 μg of pBSF2-38 (see Nature 324 73-76 (I986)), which contains the BSF-2 cDNA in the SmaI site of pGEM4, were digested with 700 units of EcoRI and BamHI in 500 μl of a high salt buffer (I00 mM NaCl, 50 mM Tris-HCl, pH 7.5, I0 mM $MgCl_2$, I mM DTT) for 2 hrs at 37°C. The digested DNA was applied on a preparative I % agarose gel electrophoresis and the EcoRI-BamHI fragment containing the full-length I.2 kbp BSF-2 cDNA was electroeluted from the gel, precipitated with 70 % ethanol and dissolved at a concentration of I μg/μl in TE buffer. 20 μg of this fragment were digested with 40 units of HinfI in 50 μl of a high salt buffer for I hr, phenol-extracted and ethanol-precipitated. The digested DNAs were dissolved in 25 μl of I ×

nick translation buffer (50 mM Tris-HCl, pH 7.2, l0 mM MgSO₄, 0.l mM DTT, 50 $\mu$g/ml BSA) and incubated with l ml of 8.2 units/$\mu$l Klenow fragment and l mM dNTP at 20°C for 30 minutes. The filling in reaction was terminated by incubation at 70°C for 5 min. The resulting l27 bp blunt ended fragment was phenol-extracted, digested with 40 units KpnI in 50 $\mu$l of a low salt buffer (l0 mM Tris-HCl, pH 7.5, l0 mM MgCl₂, l mM DTT) for l hr at 37°C, incubated with 2.5 units bacterial alkaline phosphatase at 65°C for 30 min and then applied on l % preparative agarose gel and electrophoresed. The ll0 bp fragment containing the BSF-2 leader sequence was electroeluted and ethanol-precipitated. - The ll0 bp fragment was dissolved in l0 $\mu$l of ligation buffer (50 mM Tris-HCl, pH 7.4, l0 mM MgCl₂, l0 mM DTT, l mM spermidine, l mM ATP, 0,l mg/ml BSA) and ligated with l $\mu$g of KpnI and SmaI digested pGEM4 by incubating with 200 units of T4 ligase at 4°C for l6 hrs and transfected into E.coli (MCl065). From the obtained colonies four colonies were picked up, one clone was selected, propagated and after confirmation that the plasmid of this selected clone contained only one leader sequence, it was named as pBSF2-L8 (see Figure 4).

b) 80 $\mu$g of plasmid LE-392 or pGEM4-(pFc$_\epsilon$R-l) were digested with l50 units of HindIII in 200 $\mu$l of a low salt buffer (l0 mM Tris-HCl, pH 7.5, l0 mM MgCl₂, l mM DTT) at 37°C for l hr and applied on l % preparative agarose gel electrophoresis. The HindIII fragment containing the soluble Fc$_\epsilon$R region was electroeluted from the gel, ethanol-precipitated and dissolved at a concentration of l $\mu$g/$\mu$l in TE buffer. l $\mu$g of the HindIII fragment was incubated with 8.2 units Klenow fragment and l mM dNTP in l0 $\mu$l of l × nick translation buffer at 20°C for 30 min to fill in the recessive 3'-ends, phenol-extracted and ethanol-precipitated. The HindIII fragments, the 3'-ends of which had been filled in, were digested with 2 units PstI in l0 $\mu$l of the medium salt buffer (50 mM NaCl, l0 mM Tris-HCl, pH 7.5, l0 mM MgCl₂, l mM DTT) at 37°C for l hr, incubated with 0.25 unit bacterial alkaline phosphatase at 65°C for 30 min and ethanol-precipitated. Separately, l $\mu$g of pBSF2-L8 was digested with 2 units BamHI in 20 $\mu$l of a high salt buffer at 37°C for l hr, phenol-extracted and ethanol-precipitated. The BamHI-digested pBSF2-L8 was dissolved in l0 $\mu$l of l × nick translation buffer and incubated with 8.2 units Klenow fragment and l mM dNTP at 20°C for 30 min to fill in the recessive 3'-ends, phenol-extracted and ethanol-precipitated. The precipitate was dissolved in 20 $\mu$l of a high salt buffer, digested with 2 units PstI at 37°C for l hr, phenol-extracted and ethanol-precipitated. - The PstI-digested fragment containing the soluble Fc$_\epsilon$R coding region and PstI digested pBSF2-L8 were ligated by incubating with 200 units T4 ligase in l0 $\mu$l of ligation buffer at 4°C for l6 hrs and transfected into E.coli (MCl065). Eight colonies were picked up from the obtained colonies. One clone was selected, propagated and after confirmation of the plasmid construction named psFc$_\epsilon$R-l. The propagated psFc$_\epsilon$R-l contains seven bases from the multiple cloning into pGEM4 between BSF-2 leader and Fc$_\epsilon$R sequences in frame (see Figure l: nucleotides l37 to l43).

## Example 2

### Expression of Fc$_\epsilon$R cDNA

The plasmid psFc$_\epsilon$R-l containing the modified Fc$_\epsilon$R cDNA was linearized by digestion with the restriction enzyme BamHI. mRNA was synthesized with SP6 RNA polymerase using the linearized plasmid DNA as the template according to Melton et al. About ten nanogram of mRNA was injected into each Xenopus leavis oocyte, and the oocytes were incubated at 20°C in Barth's modified medium supplemented with l00 $\mu$g/ml penicillin and l $\mu$g/ml streptomycin. After incubation for 2 days, the culture supernatant was collected and the oocytes were homogenized in Dulbecco's PBS containing l mM PMSF. The PBS-lysate was separated by high speed centrifugation at l5,000 rpm for l0 min. The pellet was again extracted with NP-40 to solubilize membrane bound receptor (0,5 % NP-40, 0,l M NaCl, 0,05 M Tris-HCl, pH 7,5).

## Example 3

### SDS-PAGE analysis of Fc$_\epsilon$R in oocytes

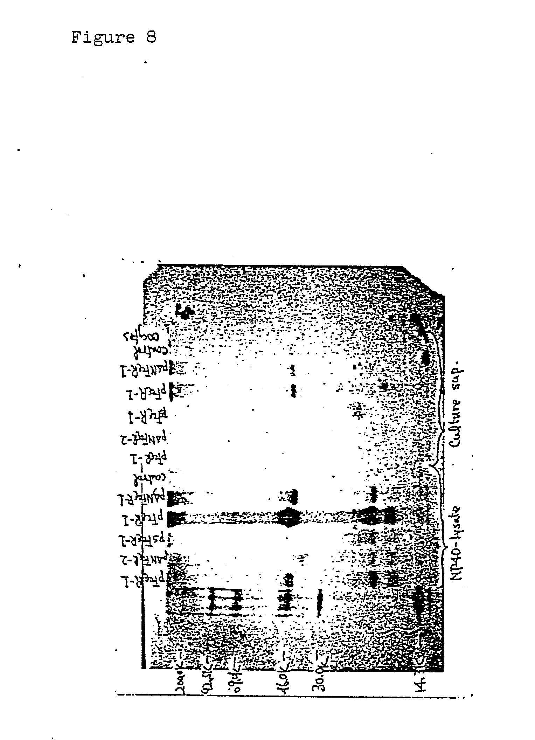

Ten oocytes which had been injected with mRNA were incubated with l00 $\mu$l Barth's modified medium containing l50 $\mu$Ci $^{35}$S-methionine for 24 hours at 20°C. Labeled oocytes were lysed in l ml lysis buffer (0,5 % NP-40, 0,l M NaCl, 0,05 M TrisHCl, pH 7.5) and centrifuged at l5,000 rpm for l0 min. The clarified lysate and culture supernatant were precleared with normal mouse Ig coupled Sepharose 4B beads and subsequently incubated with 3-5 (IgGl)antibody coupled Sepharose 4B beads for 60 min. with frequent shaking on ice. The beads were washed 2 times with lysis buffer and 2 times with high salt buffer (0,5 % NP-40, 0,5 M NaCl, 0,05 M Tris-HCl, pH 8.0) followed by a final wash with lysis buffer. The immunoprecipitates were eluted with SDS sample buffer by boiling for 2 min. Samples were analysed on 9 % SDS Page (see Figure 8).

Example 4

Detection of FcₑR

FcₑR activity was measured with a double antibody enzyme-linked immunosorbent assay (ELISA) by using two different monoclonal anti-FcₑR antibodies, 3-5(IgGl) and 8-248(IgM) which react with two different epitopes on FcₑR. Ninty six well microtiter plates (Nunc, Roskilde, Denmark) were precoated with l00 μl/well of 3-5 antibody (l0 μg/ml) in coating buffer (0,l M carbonate buffer, pH 9,6, 0,02 %), and incubated overnight at 4°C. The plates were then washed 4 times with rinse buffer (Dulbecco's phosphate buffer, pH 7,4, containing 0,05 % (v/v) Tween 20) followed by the addition of l00 μl samples diluted with diluent buffer (0,05 M Tris-HCl, pH 8,l, with l mM MgCl₂, 0,l5 M NaCl, 0,05 % Tween 20, l % BSA and 0,02 % NaN3). The plates were incubated for 2 hours at room temperature, and washed 4 times with rinse buffer, followed by the addition of l00 ml of 8-248 antibody-alkaline phosphatase conjugate (0,75 mg/ml). After 4 hours of incubation, the plates were washed 4 times and the enzyme reaction was initiated by the addition of l00 μl/well of l mg/ml p-nitrophenyl phosphate (Sigma Chemical Co., St. Louis, MO) in substrate buffer (0,05 M carbonate buffer, pH 9,8, l0 mM MgCl₂). After an appropriate incubation (60-90 min.) absorbances were read with an automatic micro-ELISA reader (Nippon Inter. Med. Tokyo, Japan) at 405 and 620 nm wavelengths (see Figures 5 and 6).

Example 5

Determination of binding of Fcₑ/R to IgE

The 3-5 antibody-coated plates were incubated with l00 μl samples for 2 hours, washed 4 times with rinse buffer, followed by the addition of l0 μg/ml human monoclonal IgE (PS myeloma). After 2 hour's incubation at room temperature, the plates were washed 4 times and incubated further with alkaline phosphataseconjugated monoclonal anti-human IgE and then developed with the addition of substrated, p-nitrophenyl phosphate as described earlier.

Example 6

IgE rosette formation

FcₑR on lymphocytes are detected by an assay with the use of fixed ox RBC (ORBC) coated with human IgE (Gonzalez-Molina, A. and Spiegelberg, H.L. J. Clin. Invest 59, 6l6 (l977)). The number of IgE rosette-forming cells is estimated after subtracting the number of non-specific binding with fixed ORBC coated with bovine serum albumin. For IgE rosette inhibition, 25 μl of FcₑR bearing cells (5 × l0⁶/ml) are mixed with a specific volume (e.g. l00 μl) of test sample or control medium and incubated for l hour at 4°C. The number of rosettes with 3 or more ORBC are counted. In experiments I and III the FcₑR bearing cells are RPMl8866 cells and in experiment II SKW6-CL4 cells. The control medium is the supernatant of the control oocytes, i.e. non-transformed oocytes.

In the foregoing test results it will be seen, a) that SDS-PAGE analysis shows the product of psFcₑR-l from oocytes, which is recognized by both antibodies 3-5 and 8-30 and has IgE-binding activity, yielded broad protein bands (see Figure 8) and, b) the product of pΔNFcₑR-l from oocytes, which by comparison lacks the N-terminal transmembrane region, can be recognized by the 3-5 antibody, but not by the 8-30 antibody and does not have IgE binding activity. These results indicate that the product of pΔNFcₑR-l which does not have a signal sequence is not processed properly and thus that a proper processing as in clone psFcₑR-l create the epitope which is recognized by the 8-30 antibody and has IgE binding activity.

**Claims**

l. Recombinant biologically active water-soluble fragment of human FcₑR having at least one O-glycosylation site, preferably a fragment accompanied by native O-glycosylation.

2. O-glycosylated water-soluble fragment of FcₑR as claimed in claim l characterized by the DNA-sequence of the fulllength FcₑR cDNA, wherein at least a part of the coding sequence for the amino acids l to l48 as shown in Figure l is replaced by an eucaryotic signal sequence, or a degenerative derivate thereof.

3. O-glycosylated water-soluble fragment of FcₑR as claimed in claim l or in claim 2 containing the amino acids l50 to 32l as shown in Figure l.

4. O-glycosylated water-soluble fragment of FcₑR as claimed in claim 2 wherein the signal sequence of pBSF-2.38 is used as eucaryotic signal sequence, or a degenerative derivate thereof.

5. O-glycosylated water-soluble fragment of FcₑR as claimed in claim 4 containing the amino acids 55 to 226 as shown in Figure 3 accompanied by native O-glycosylation, or a degenerative derivate thereof.

6. A recombinant DNA molecule which contains the genetic information for producing a water-soluble fragment of human $Fc_\epsilon R$ accompanied by native O-glycosylation as claimed in any of the claims I to 5, or a degenerative derivate thereof.

7. A recombinant DNA molecule as claimed in claim 6 containing in addition the replicon and control sequences necessary for expression.

8. A recombinant DNA molecule as claimed in claim 7 containing the replicon and control sequences for expression in eukaryotes, especially in mammalian cells.

9. A recombinant DNA molecule as claimed in claim 7 or 8 containing the replicon and control sequences suitable for expression in yeast.

I0. A recombinant DNA molecule as claimed in claim 9 wherein as replicon and control sequences those of plasmid pGEM4 are used.

II. A vector containing a recombinant DNA molecule as claimed in any of the claim 6 to I0.

I2. A vector as claimed in claim II wherein the vector is a plasmid vector.

I3. A plasmid as claimed in claim I2 containing the coding sequence as claimed in any of the claims 6 to I0.

I4. $psFc_\epsilon R$-I as shown in Figure 3 or a degenerative derivate thereof.

I5. A host organism transformed by a vector as claimed in any of the claims II to I4.

I6. Pharmaceutical compositions containing a polypeptide as claimed in any of the claims I to 5.

I7. Pharmaceutical compositions as claimed in claim I6 suitable for treatment of local or systemic IgE-allergic reactions.

I8. Preparation of a pharmaceutical composition as claimed in claims I6 or I7 wherein an effective amount of a polypeptide as claimed in any of the claims I to 5 is incorporated in one or more excipients.

I9. Use of a polypeptide as claimed in any of the claims I to 5 for the preparation of a pharmaceutical composition.

20. A process for preparing an O-glycosylated active water-soluble fragment of human $Fc_\epsilon R$ as claimed in any of the claims I to 5 which comprises transforming a suitable host organism by a vector containing a DNA as claimed in any of the claim 6 to I0 for the desired fragment of $Fc_\epsilon R$ at an appropriate site for expression, and isolating the secreted fragment from the resulting transformants.

2I. Process for preparing a host organism as claimed in claim I5, wherein a suitable host is transformed with a vector as claimed in any of the claims II to I4.

22. Process for preparing a vector as claimed in any of the claims II to I4, wherein a DNA as claimed in any of the claims 6 to I0 is inserted in a suitable vector.

23. Process for preparing a DNA as claimed in any of the claims 6 to I0, wherein a suitable vector is digested with one or more suitable restriction endonucleases and isolating the desired DNA.

Figure 1: Scheme of pFc<sub>ε</sub>R-1

EcoRI

——————————————————————————————— GAATTCCCTCCTGCT   8

TAAACCTCTGTCTCTGACGGTCCCTGCCAATCGCTCTGGTCGACCCCAACACACTAGGA   67

GGACAGACACAGGCTCCAAACTCCACTAAGTGACCAGAGCTGTGATTGTGCCCGCTGAG   126

TGGACTGCGTTGTCAGGGAGTGAGTGCTCCATCATCGGGAGAATCCAAGCAGGACCGCC   185

```
                      5                 10                15
    Met Glu Glu Gly Gln Tyr Ser Glu Ile Glu Glu Leu Pro Arg Arg
    ATG GAG GAA GGT CAA TAT TCA GAG ATC GAG GAG CTT CCC AGG AGG   230

                      20                25                30
    Arg Cys Cys Arg Arg Gly Thr Gln Ile Val Leu Leu Gly Leu Val
    CGG TGT TGC AGG CGT GGG ACT CAG ATC GTG CTG CTG GGG CTG GTG   275

                      35                40                45
    Thr Ala Ala Leu Trp Ala Gly Leu Leu Thr Leu Leu Leu Leu Trp
    ACC GCC GCT CTG TGG GCT GGG CTG CTG ACT CTG CTT CTC CTG TGG   320

                      50                55                60
    His Trp Asp Thr Thr Gln Ser Leu Lys Gln Leu Glu Glu Arg Ala
    CAC TGG GAC ACC ACA CAG AGT CTA AAA CAG CTG GAA GAG AGG GCT   365

                      65                70                75
    Ala Arg Asn Val Ser Gln Val Ser Lys Asn Leu Glu Ser His His
    GCC CGG AAC GTC TCT CAA GTT TCC AAG AAC TTG GAA AGC CAC CAC   410

                      80                85                90
    Gly Asp Gln Met Ala Gln Lys Ser Gln Ser Thr Gln Ile Ser Gln
    GGT GAC CAG ATG GCG CAG AAA TCC CAG TCC ACG CAG ATT TCA CAG   455

                      95                100               105
    Glu Leu Glu Glu Leu Arg Ala Glu Gln Gln Arg Leu Lys Ser Gln
    GAA CTG GAG GAA CTT CGA GCT GAA CAG CAG AGA TTG AAA TCT CAG   500

                      110               115               120
    Asp Leu Glu Leu Ser Trp Asn Leu Asn Gly Leu Gln Ala Asp Leu
    GAC TTG GAG CTG TCC TGG AAC CTG AAC GGG CTT CAA GCA GAT CTG   545

                      125               130               135
    Ser Ser Phe Lys Ser Gln Glu Leu Asn Glu Arg Asn Glu Ala Ser
    AGC AGC TTC AAG TCC CAG GAA TTG AAC GAG AGG AAC GAA GCT TCA   590

                      140               145               150
    Asp Leu Leu Glu Arg Leu Arg Glu Glu Val Thr Lys Leu Arg Met
    GAT TTG CTG GAA AGA CTC CGG GAG GAG GTG ACA AAG CTA AGG ATG   635

                      155               160               165
    Glu Leu Gln Val Ser Ser Gly Phe Val Cys Asn Thr Cys Pro Glu
    GAG TTG CAG GTG TCC AGC GGC TTT GTG TGC AAC ACG TGC CCT GAA   680
```

```
                 170                   175                   180
Lys Trp Ile Asn Phe Gln Arg Lys Cys Tyr Tyr Phe Gly Lys Gly
AAG TGG ATC AAT TTC CAA CGG AAG TGC TAC TAC TTC GGC AAG GGC   725

                 185                   190                   195
Thr Lys Gln Trp Val His Ala Arg Tyr Ala Cys Asp Asp Met Glu
ACC AAG CAG TGG GTC CAC GCC CGG TAT GCC TGT GAC GAC ATG GAA   770

                 200                   205                   210
Gly Gln Leu Val Ser Ile His Ser Pro Glu Glu Gln Asp Phe Leu
GGG CAG CTG GTC AGC ATC CAC AGC CCG GAG GAG CAG GAC TTC CTG   815

                 215                   220                   225
Thr Lys His Ala Ser His Thr Gly Ser Trp Ile Gly Leu Arg Asn
ACC AAG CAT GCC AGC CAC ACC GGC TCC TGG ATT GGC CTT CGG AAC   860

                 230                   235                   240
Leu Asp Leu Lys Gly Glu Phe Ile Trp Val Asp Gly Ser His Val
TTG GAC CTG AAG GGA GAG TTT ATC TGG GTG GAT GGG AGC CAT GTG   905

                 245                   250                   255
Asp Tyr Ser Asn Trp Ala Pro Gly Glu Pro Thr Ser Arg Ser Gln
GAC TAC AGC AAC TGG GCT CCA GGG GAG CCC ACC AGC CGG AGC CAG   950

                 260                   265                   270
Gly Glu Asp Cys Val Met Met Arg Gly Ser Gly Arg Trp Asn Asp
GGC GAG GAC TGC GTG ATG ATG CGG GGC TCC GGT CGC TGG AAC GAC   995

                 275                   280                   285
Ala Phe Cys Asp Arg Lys Leu Gly Ala Trp Val Cys Asp Arg Leu
GCC TTC TGC GAC CGT AAG CTG GGC GCC TGG GTG TGC GAC CGG CTG  1040

                 290                   295                   300
Ala Thr Cys Thr Pro Pro Ala Ser Glu Gly Ser Ala Glu Ser Met
GCC ACA TGC ACG CCG CCA GCC AGC GAA GGT TCC GCG GAG TCC ATG  1085

                 305                   310                   315
Gly Pro Asp Ser Arg Pro Asp Pro Asp Gly Arg Leu Pro Thr Pro
GGA CCT GAT TCA AGA CCA GAC CCT GAC GGC CGC CTG CCC ACC CCC  1130

                 320
Ser Ala Pro Leu His Ser   *
TCT GCC CCT CTC CAC TCT TGA GCATGGATACAGCCAGGCCCAGAGCAAGACC  1182

CTGAAGACCCCCAACCACGGCCTAAAAGCCTCTTTGTGGCTGAAAGGTCCCTGTGACAT  1241

TTTCTGCCACCCAAACGGAGGCAGCTGACACATCTCCCGCTCCTCTATGGCCCCTGCCT  1300
```

```
TCCCAGGAGTACACCCCAACAGCACCCTCTCCAGATGGGAGTGCCCCCAACAGCACCCT 1359

CTCCAGATGAGAGTACACCCCAACAGCACCCTCTCCAGATGCAGCCCCATCTCCTCAGC 1418

ACCCCAGGACCTGAGTATCCCCAGCTCAGGTGGTGAGTCCTCCTGTCCAGCCTGCATCA 1477

ATAAAATGGGGCAGTGATGGCCTCCCAAAAA -------------------------- 1507

----- AAGGAATTC /Sac/Kpn/    /Pst/Sph/Hind/
              EcoRI
```

0 286 700

Figure 2

(1) pFcεR-1

(2) pΔN-FcεR-1

(3) pΔN-FcεR-2

(4) psFcεR-1

Figure 3: Scheme of psFcₑR-1

——————————————————————————————ATTTAGGTGACACTATA

```
                                          2                        7
                                        Met Asn Ser Phe Ser Thr Ser
      EcoRI          Kpnl
   GAATACACGGAATTCGAGCTCGGTACCCCT ATG AAC TCC TTC TCC ACA ACC    51

                  12                    17                   22
   Ala Phe Gly Pro Val Ala Phe Ser Leu Gly Leu Leu Leu Val Leu
   GCC TTC GGT CCA GTT GCC TTC TCC CTG GGG CTG CTC CTG GTG TTG    96

                  27                    32                   37
   Pro Ala Ala Phe Pro Ala Pro Val Pro Pro Gly Glu Asp Trp Gly
   CCT GCT GCC TTC CCT GCC CCA GTA CCC CCA GGA GAA GAT TGG GGA   141

                  42                    47                   52
   Ser Ala Ser Asp Leu Leu Glu Arg Leu Arg Glu Glu Val Thr Lys
   TCA GCT TCA GAT TTG CTG GAA AGA CTC CGG GAG GAG GTG ACA AAG   186

                  57                    62                   67
   Leu Arg Met Glu Leu Gln Val Ser Ser Gly Phe Val Cys Asn Thr
   CTA AGG ATG GAG TTG CAG GTG TCC AGC GGC TTT GTG TGC AAC ACG   231

                  72                    77                   82
   Cys Pro Glu Lys Trp Ile Asn Phe Gln Arg Lys Cys Tyr Tyr Phe
   TGC CCT GAA AAG TGG ATC AAT TTC CAA CGG AAG TGC TAC TAC TTC   276

                  87                    92                   97
   Gly Lys Gly Thr Lys Gln Trp Val His Ala Arg Tyr Ala Cys Asp
   GGC AAG GGC ACC AAG CAG TGG GTC CAC GCC CGG TAT GCC TGT GAC   321

                  102                   107                  112
   Asp Met Glu Gly Gln Leu Val Ser Ile His Ser Pro Glu Glu Gln
   GAC ATG GAA GGG CAG CTG GTC AGC ATC CAC AGC CCG GAG GAG CAG   366

                  117                   122                  127
   Asp Phe Leu Thr Lys His Ala Ser His Thr Gly Ser Trp Ile Gly
   GAC TTC CTG ACC AAG CAT GCC AGC CAC ACC GGC TCC TGG ATT GGC   411

                  132                   137                  142
   Leu Arg Asn Leu Asp Leu Lys Gly Glu Phe Ile Trp Val Asp Gly
   CTT CGG AAC TTG GAC CTG AAG GGA GAG TTT ATC TGG GTG GAT GGG   456

                  147                   152                  157
   Ser His Val Asp Tyr Ser Asn Trp Ala Pro Gly Glu Pro Thr Ser
   AGC CAT GTG GAC TAC AGC AAC TGG GCT CCA GGG GAG CCC ACC AGC   501

                  162                   167                  172
   Arg Ser Gln Gly Glu Asp Cys Val Met Met Arg Gly Ser Gly Arg
   CGG AGC CAG GGC GAG GAC TGC GTG ATG ATG CGG GGC TCC GGT CGC   546

                  177                   182                  187
   Trp Asn Asp Ala Phe Cys Asp Arg Lys Leu Gly Ala Trp Val Cys
   TGG AAC GAC GCC TTC TGC GAC CGT AAG CTG GGC GCC TGG GTG TGC   591
```

```
              192                  197                  202
Asp Arg Leu Ala Thr Cys Thr Pro Pro Ala Ser Glu Gly Ser Ala
GAC CGG CTG GCC ACA TGC ACG CCG CCA GCC AGC GAA GGT TCC GCG   636

              207                  212                  217
Glu Ser Met Gly Pro Asp Ser Arg Pro Asp Pro Asp Gly Arg Leu
GAG TCC ATG GGA CCT GAT TCA AGA CCA GAC CCT GAC GGC CGC CTG   681

              222                  227
Pro Thr Pro Ser Ala Pro Leu His Ser  *
CCC ACC CCC TCT GCC CCT CTC CAC TCT TGA GCATGGATACAGCCAGGCC   730

CAGAGCAAGACCCTGAAGACCCCCAACCACGGCCTAAAAGCCTCTTTGTGGCTGAAAGG   789

TCCCTGTGACATTTTCTGCCACCCAAACGGAGGCAGCTGACACATCTCCCGCTCCTCTA   848

TGGCCCCTGCCTTCCCAGGAGTACACCCCAACAGCACCCTCTCCAGATGGGAGTGCCCC   907

CAACAGCACCCTCTCCAGATGAGAGTACACCCCAACAGCACCCTCTCCAGATGCAGCCC   966

CATCTCCTCAGCACCCCAGGACCTGAGTATCCCCAGCTCAGGTGGTGAGTCCTCCTGTC   1025

CAGCCTGCATCAATAAAATGGGGCAGTGATGGCCTCCCAAAAAAAA ------------  1071

- AAAAGGAATTCGAGCTCGGTACCCGGGGATCCTCTAGAGTCGACCTGCAGGCATGCA

AGCTTCCGGTCTCCCTATAGTGAGTCCTATTA
```

## Figure 4: Scheme of pBSF2-L8

```
                                          -25
                          KpnI   Met Asn Ser Phe
              /Eco/Sac/GGTACC ATG AAC TCC TTC    18

          -20                      -15              -10
Ser Thr Ser Ala Phe Gly Pro Val Ala Phe Ser Leu Gly Leu Leu
TCC ACA AGC GCC TTC GGT CCA GTT GCC TTC TCC CTG GGG CTG CTC    63

               -5             1                    6
Leu Val Leu Pro Ala Ala Phe Pro Ala Pro Val Pro Pro Gly Glu
CTG GTG TTG CCT GCT GCC TTC CCT GCC CCA GTA CCC CCA GGA GAA    108

          11                   16                   21
Asp Ser Lys Asp Val Ala Ala Pro His Arg Gln Pro Leu Thr Ser
GAT TCC AAA GAT GTA GCC GCC CCA CAC AGA CAG CCA CTC ACC TCT    153

          26                   31                   36
Ser Glu Arg Ile Asp Lys Gln Ile Arg Tyr Ile Leu Asp Gly Ile
TCA GAA CGA ATT GAC AAA CAA ATT CGG TAC ATC CTC GAC GGC ATC    198

          41                   46                   51
Ser Ala Leu Arg Lys Glu Thr Cys Asn Lys Ser Asn Met Cys Glu
TCA GCC CTG AGA AAG GAG ACA TGT AAC AAG AGT AAC ATG TGT GAA    243

          56                   61                   66
Ser Ser Lys Glu Ala Leu Ala Glu Asn Asn Leu Asn Leu Pro Lys
AGC AGC AAA GAG GCA CTG GCA GAA AAC AAC CTG AAC CTT CCA AAG    288

          71                   76                   81
Met Ala Glu Lys Asp Gly Cys Phe Gln Ser Gly Phe Asn Glu Glu
ATG GCT GAA AAA GAT GGA TGC TTC CAA TCT GGA TTC AAT GAG GAG    333

          86                   91                   96
Thr Cys Leu Val Lys Ile Ile Thr Gly Leu Leu Glu Phe Glu Val
ACT TGC CTG GTG AAA ATC ATC ACT GGT CTT TTG GAG TTT GAG GTA    378

          101                  106                  111
Tyr Leu Glu Tyr Leu Gln Asn Arg Phe Glu Ser Ser Glu Glu Gln
TAC CTA GAG TAC CTC CAG AAC AGA TTT GAG AGT AGT GAG GAA CAA    423

          116                  121                  126
Ala Arg Ala Val Gln Met Ser Thr Lys Val Leu Ile Gln Phe Leu
GCC AGA GCT GTG CAG ATG AGT ACA AAA GTC CTG ATC CAG TTC CTG    468

          131                  136                  141
Gln Lys Lys Ala Lys Asn Leu Asp Ala Ile Thr Thr Pro Asp Pro
CAG AAA AAG GCA AAG AAT CTA GAT GCA ATA ACC ACC CCT GAC CCA    513

          146                  151                  156
Thr Thr Asn Ala Ser Leu Leu Thr Lys Leu Gln Ala Gln Asn Gln
ACC ACA AAT GCC AGC CTG CTG ACG AAG CTG CAG GCA CAG AAC CAG    558
```

```
                     161                        166                        171
Trp Leu Gln Asp Met Thr Thr His Leu Ile Leu Arg Ser Phe Lys
TGG CTG CAG GAC ATG ACA ACT CAT CTC ATT CTG CGC AGC TTT AAG   603

                     176                     181
Glu Phe Leu Gln Ser Ser Leu Arg Ala Leu Arg Gln Met
GAG TTC CTG CAG TTC AGC CTG AGG GCT CTT CGG CAA ATG TAGCATG   649

GGCACCTCAGATTGTTGTTGTTAATGGGCATTCCTTCTTCTGGTCAGAAACCTGTCCAC   708

TGGGCACAGAACTTATGTTGTTCTCTATGGAGAACTAAAAGTATGAGCGTTAGGACACT   767

ATTTTAATTATTTTTAATTTATTAATATTTAAATATGTGAAGCTGAGTTAATTTATGTA   826

AGTCATATTTATATTTTAAGAAGTACCACTTGAAACATTTTATGTATTAGTTTTGAAAT   885

AATAATGGAAAGTGGCTATGCAGTTTGAATATCCTTTGTTTCAGAGCCAGATCATTTCT   944

TGGAAAGTGTAGGCTTACCTCAAATAAATGGCTAACTTATACATATTTTTAAAGAAATA  1003

TTTATATTGTATTTATATAATGTATAAATGGTTTTTATACCAATAAATGGCATTTTAAA  1062

AAATTCAGCAAAAAAAAAAAAAAAAAAAAAAAAAGGGATCC/Xba/SaI/Pst/Sph/Hin/1100
                                          BamHI
```

Figure 5

unit

1.0

0.5

pFc$_\varepsilon$R-1    p$\Delta$N-Fc$_\varepsilon$R-1    p$\Delta$N-Fc$_\varepsilon$R-2    psFc$_\varepsilon$R-1

☐ PBS-lysate

▨ Np40-lysate

■ supernatant

0 286 700

Figure 6

Figure 7

(No. of ORBC bound to cells)

0 286 700

Figure 8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | THE EMBO JOURNAL, vol. 6, no. 1, January 1987, pages 109-114, IRL Press Ltd, Eynsham, Oxford, GB; C. LÜDIN et al.: "Cloning and expression of the cDNA coding for a human lymphocyte IgE receptor" * Whole document * | 1,6-10 | C 12 N 15/00 C 07 K 13/00 A 61 K 37/02 C 12 P 21/00 |
| Y | Idem --- | 2-5 | |
| Y,D | NATURE, vol. 324, 6th November 1986, pages 73-77; T. HIRANO et al.: "Complementary DNA for a novel human interleukin (BSF-2) that induces B lymphocytes to produce immunoglobulin" * Figure 3 * --- | 2-5 | |
| X,D | CELL, vol. 47, December 1986, pages 657-665; H. KIKUTANI et al.: "Molecular structure of human lymphocyte receptor for immunoglobulin E" * Whole document * --- | 1,6-10 | |
| E | EP-A-0 248 211 (J. YODOI) * Whole document * ----- | 1,6-10 | TECHNICAL FIELDS SEARCHED (Int. Cl.4) C 12 N A 61 K |

The present search report has been drawn up for all claims

| Place of search | Date of completion of the search | Examiner |
|---|---|---|
| THE HAGUE | 11-12-1987 | CUPIDO M. |

European Patent
Office

## CLAIMS INCURRING FEES

The present European patent application comprised at the time of filing more than ten claims.

☐ All claims fees have been paid within the prescribed time limit. The present European search report has been drawn up for all claims.

☐ Only part of the claims fees have been paid within the prescribed time limit. The present European search report has been drawn up for the first ten claims and for those claims for which claims fees have been paid,

namely claims:

☒ No claims fees have been paid within the prescribed time limit. The present European search report has been drawn up for the first ten claims.

## LACK OF UNITY OF INVENTION

The Search Division considers that the present European patent application does not comply with the requirement of unity of invention and relates to several inventions or groups of inventions,
namely:

☐ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid,

namely claims:

☐ None of the further search fees has been paid within the fixed time limit. The present European search report has been drawn up for those parts of the European patent application which relate to the invention first mentioned in the claims,

namely claims: